# EUROPEAN PATENT SPECIFICATION

(11) **EP 0 581 450 B1**
(45) Date of publication and mention of the grant of the patent: **25.09.1996**
(21) Application number: 93305148.4
(22) Date of filing: 30.06.1993
(51) Int. Cl.: A61B 5/00

(54) **Monitoring of the condition of a fetus during labour**
Überwachung des fötalen Gesundheitszustands während der Wehen
Surveillance de l'état d'un foetus pendant l'accouchement

(30) Priority: 01.07.1992 GB 9213987
(43) Date of publication of application: 02.02.1994
(73) Proprietor: BRITISH TECHNOLOGY GROUP LIMITED, London SE1 6BU (GB)
(72) Inventor: Genevier, Eric, London NW10 5JG (GB); Steer, Philip James, Kingston upon Thames, Surrey KT2 7BJ (GB); Danielian, Peter James, London SW2 5BM (GB)
(74) Representative: Bird, Christopher John

(56) References cited:
- EP-A- 0 323 816
- WO-A-92/00699
- FR-A- 2 608 909
- SENSORS AND ACTUATORS B CHEMICAL vol. B7, no. 1/3 , March 1992 , LAUSANNE, CH, pages 775 - 779; BECHI ET AL. 'Optical fibre bile sensor for entero-gastric reflux detection'

## Description

This invention relates to the monitoring of the condition of a fetus during labour. In our PCT application Publication No. WO 92/00699, we describe a method and system for monitoring the quality of amniotic fluid (AF) during labour. Experience with the system described in our prior application has demonstrated the importance of excluding any light which is scattered by the uterine wall or the skin of the fetus. Such randomly scattered light tends to give a spurious signal indicative of the presence of blood because the uterine wall is richly supplied with blood vessels. Also, it has been found that it is advantageous to design the probe so that amniotic fluid flows freely to and through the optical cell.

According to one aspect of the present invention there is provided an intrauterine probe which comprises a flexible body housing an optical cell, said cell including at least one aperture permitting entry of amniotic fluid and being connected at one end to a fibre optic cable for conducting light reflected from the amniotic fluid to spectral analysis means, said cell also having shielding means to shield the fibre optic cable from any light scattered by the wall of the uterus or the fetus.

Preferably, the probe has a flattened form and is designed so that the generally flat faces of the probe contact the fetus on one side and the wall of the uterus on the other. With such a design, the entry to the optical cell for amniotic fluid is via the sides which are generally at right angles to the flattened faces of the probe and the optical cell within the probe is shielded by flattened portions of the sides of the probe. It is also preferred that the optical cell has at least two apertures so that amniotic fluid can flow freely through the optical cell.

One embodiment of the present invention will now be described with reference to the accompanying drawings, in which:-
Figure 1 is a general arrangement showing the layout of the system;
Figure 2A is a side elevation of the tip of the probe;
Figure 2B is an end view of the tip of the probe; and
Figure 2C is a view of the tip of the probe seen in the direction of the arrow A in Figure 2A.

The system layout is similar to that described in our above PCT application and incorporates a flexible probe 1 which is linked by fibre optic cables 2 and 3 respectively to a broad spectrum light source (4) such as a tungsten halogen lamp or a xenon arc lamp and to a spectral analysis equipment. A xenon arc lamp is preferred since it is rich in radiation around the 405-415 nm band which includes the meconium absorption peak.

Fibre optic cable 3 is connected to spectral analysis equipment 5 for measuring back scattered light from the amniotic fluid. A typical system is the Monolight Optical Spectrum Analyser System 6800, manufactured by Monolight Limited of Weybridge, Surrey. This system consists of a diffraction grating with an input slit width of 0.89 mms achieving a band width resolution of 10 nm and has a photomultiplier tube with a variable output gain and a 12 bit analogue to digital converter. The converter is connected to an IBM PC (6) and the results displayed on the screen. The spectra of the back scattered light were measured in the range of 300 to 900 nm. The probe may also include a pressure sensor and/or electrodes, e.g. as described in UK Patents Nos. 2195897 and 2216904 and EP No. 0325605, and the data collected, i.e. fetal heart rate and intrauterine pressure displayed on the screen or on a separate monitor 7.

The fibre optic cables 2 and 3 were merged together in the body of the probe 1 to form a merged bundle 10 which terminated at one end of the optical cell 11. The other end 12 of the optical cell opposite to the ends of the fibre optic cable constitutes a "beam dump" and forms a cavity having sides forming an approximate right angle. This shape and the black colouration of the probe body ensures that light striking end 12 of the cell, e.g. light entering from the transmission part of the fibre optic bundle is efficiently absorbed and not reflected back into the receptor fibres of the cable.

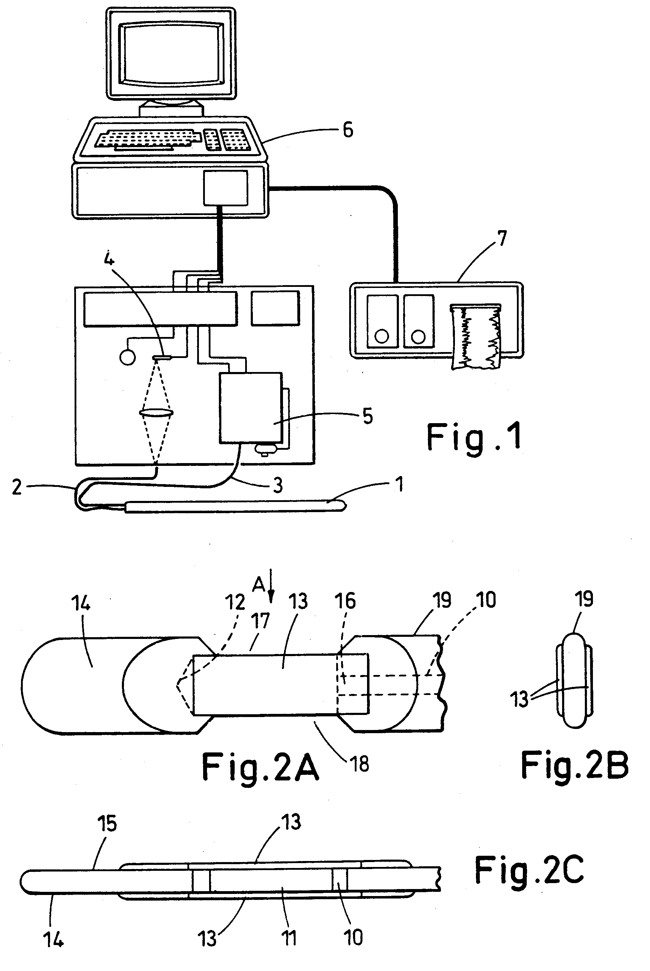

As shown, the sides of the optical cell comprise a pair of strip-like shielding members 13 which ensure that any light reflected from the uterine wall against which the face 14 or 15 may be pressed is not reflected into the end of the fibre optic bundle 16. In the embodiment illustrated the members 13 are formed separately from the body of the probe and are bonded to the distal end and the main body of the probe to form the optical cell. Alternatively, of course, the parts 13 may be constructed integrally with the body of the probe (e.g. as a moulding or extrusion). It is, however, thought to be advantageous that the entry to the apertures 17 and 18 are recessed from the general upper surface 19 of the probe body. It is believed that in the uterus during labour the amniotic fluid may be trapped in pools between portions of the uterine wall and the fetus and the design shown in Figures 2A to 2C tends to encourage amniotic fluid to flow along the surfaces 19 of the probe and into the optical cell.

Half the fibres in the silica optic fibre bundle of approximately 1000 fibres were randomly chosen for transmitting light and the others to receive light back scattered from the amniotic fluid. The fibre bundle was encapsulated in the body of the intrauterine probe with its tip 16 emerging at one end of the optical cell 11, approximately 50 mms from the tip of the probe.

The thickness of the probe body was approximately 4 mms and was made from polyurethane with a total length of about 400 mms and a width of about 14 mms.

The thickness of the shielding parts 13 was about 1 mm and in the design shown in the attached drawings was formed from hard PVC so as to be stiff and not to allow significant distortion of the cell during use. The cell itself was approximately 1 cm long.

Other features of the probe may be as described in our above PCT application.

## Claims

1. An intrauterine probe (1) which comprises a flexible body which houses an optical cell (11), said cell (11) including at least one aperture (17 and/or 18) permitting the entry of amniotic fluid (AF) and being connected at one end to a fibre optic cable (3), for conducting light reflected from the AF to spectral analysis means (5), said cell (11) having shielding means (13) to shield the fibre optic cable (3) from any light scattered by the wall of the uterus or the fetus.

2. A probe (1) according to claim 1 in which the probe (1) has a generally flattened form so that in use one of the flat faces (14, 15) of the probe contacts the wall of the uterus.

3. A probe (1) according to claim 2 in which the shielding means (13) are generally planar with the faces (14, 15) of the probe.

4. A probe (1) according to claim 2 or claim 3 in which the generally flat faces (14, 15) of the probe include one or more longitudinal ribs.

5. A probe (1) according to any one of the preceding claims in which the aperture (17, 18) opens into a surface of the probe which in use faces away from the uterine wall.

6. A probe (1) according to any one of the preceding claims in which there are at least two apertures (17, 18) thereby facilitating a flow of amniotic fluid through the cell (11).

7. A system for monitoring the content of amniotic fluid during labour which comprises a probe (1) as claimed in any one of the preceding claims which is connected optically to a source of light (4) having a suitable spectral bandwidth, and to photodetecting means (5) for detecting the spectral response of the amniotic fluid (AF) to illumination with said source (4) and processing means (6) for determining the presence of meconium and/or blood in the amniotic fluid by analysis of the spectral response.

## Patentansprüche

1. Eine Intrauterinsonde (1), die einen biegsamen Körper aufweist, der eine optische Zelle (11) beherbergt, wobei die Zelle (11) mindestens eine Öffnung (17 und/oder 18) enthält, die den Eintritt eines amniotischen Fluids (AF) gestattet und an einem Ende mit einem Lichtwellenleiter (3) verbunden ist, um von dem AF reflektiertes Licht zu einem Spektralanalysemittel (5) zu leiten, wobei die Zelle (11) Abschirmmittel (13) aufweist, um den Lichtwellenleiter (3) von einem, durch die Wand des Uterus oder des Fötus gestreuten Licht abzuschirmen.

2. Eine Sonde (1) nach Anspruch 1, worin die Sonde (1) eine im allgemeinen flache Form hat, so daß bei Gebrauch eine der flachen Seiten (14, 15) der Sonde die Wand des Uterus berührt.

3. Eine Sonde (1) nach Anspruch 2, worin die Abschirmmittel (13) im allgemeinen mit den Flächen (14, 15) der Sonde eben sind.

4. Eine Sonde (1) nach Anspruch 2 oder Anspruch 3, worin die im allgemeinen flachen Flächen (14, 15) der Sonde eine oder mehr Längsrippen enthalten.

5. Eine Sonde (1) nach einem der vorhergehenden Ansprüche, worin sich die Öffnung (17, 18) in eine Oberfläche der Sonde öffnet, die bei Gebrauch von der Uteruswand abgewandt ist.

6. Eine Sonde (1) nach einem der vorhergehenden Ansprüche, worin es mindestens zwei Öffnungen (17, 18) gibt, wodurch ein Strom eines amniotischen Fluids durch die Zelle (11) erleichtert wird.

7. Ein System zum Überwachen des Inhalts eines amniotischen Fluids während der Wehen, das eine Sonde (1) nach einem der vorhergehenden Ansprüche, die mit einer Lichtquelle (4) mit einer geeigneten spektralen Bandbreite und mit einem Photodetektionsmittel (5) zum Detektieren der spektralen Empfindlichkeit des amniotischen Fluids (AF) auf eine Beleuchtung mit der Quelle (4) hin optisch verbunden ist, und ein Verarbeitungsmittel (6) zum Bestimmen des Vorhandenseins von Mekonium und/oder Blut in dem amniotischen Fluid durch eine Analyse der spektralen Empfindlichkeit aufweist.

## Revendications

1. Sonde intra-utérine (1), qui comprend un corps flexible qui loge une cellule optique (11), ladite cellule (11) comprenant au moins une ouverture (17 et/ou 18) permettant l'entrée du liquide amniotique (AF) et étant raccordée, par une extrémité, à un câble à fibre optique (3), pour envoyer la lumière réfléchie par le liquide AF à des moyens d'analyse spectrale (5), ladite cellule (11) comportant des moyens de blindage (13) servant à protéger le câble à fibre optique (3) vis-à-vis de toute lumière dispersée par la paroi de l'utérus ou du foetus.

2. Sonde (1) selon la revendication 1, dans laquelle la sonde (1) possède une forme générale aplatie de sorte que, lors de l'utilisation, l'une des faces planes (14,15) de la sonde est en contact avec la paroi de l'utérus.

3. Sonde (1) selon la revendication 2, dans laquelle les moyens de blindage (13) possèdent une forme générale plane de niveau avec les faces (14,15) de la sonde (1).

4. Sonde (1) selon la revendication 2 ou 3, dans laquelle les faces de forme générale plane (14,15) de la sonde comprennent une ou plusieurs nervures longitudinales.

5. Sonde (1) selon l'une quelconque des revendications précédentes, dans laquelle l'ouverture (17,18) débouche dans une surface de la sonde qui, en fonctionnement, est tournée à l'opposé de la paroi utérine.

6. Sonde (1) selon l'une quelconque des revendications précédentes, dans laquelle au moins deux ouvertures (17,18) sont prévues, ce qui facilite un écoulement du liquide amniotique à travers la cellule (11).

7. Système pour contrôler le contenu de liquide amniotique pendant le travail, qui comprend une sonde (1) telle que revendiquée selon l'une quelconque des revendications précédentes et qui est connectée optiquement à une source de lumière (4) possédant une largeur de bande spectrale appropriée, et à des moyens photodétecteurs (5) pour détecter la réponse spectrale du liquide amniotique (AF) à un éclairement par ladite source (4), et des moyens de traitement (6) pour déterminer la présence de méconium et/ou de sang dans le liquide amniotique au moyen de l'analyse de la réponse spectrale.
